# EUROPEAN PATENT SPECIFICATION

(11) **EP 3 697 891 B1**
(45) Date of publication and mention of the grant of the patent: **27.03.2024**
(21) Application number: 18819222.3
(22) Date of filing: 16.10.2018
(51) Int. Cl.: C12N 1/12, C12P 1/00, C12P 39/00, C12M 1/00, A01G 33/00, C12M 1/34

(54) **METHOD AND SYSTEM FOR THE ENHANCEMENT OF PRODUCTIVITY OF HYDROGENOTROPHIC MICRO-ORGANISMS**
VERFAHREN UND SYSTEM ZUR STEIGERUNG DER PRODUKTIVITÄT HYDROGENOTROPHER MIKROORGANISMEN
PROCÉDÉ ET SYSTÈME POUR L'AMÉLIORATION DE LA PRODUCTIVITÉ DE MICROORGANISMES HYDROGÉNOTROPHES

(30) Priority: 16.10.2017 NL 2019733
(43) Date of publication of application: 26.08.2020
(73) Proprietor: Avecom NV, 9032 Wondelgem (BE)
(72) Inventor: VERSCHOOR, Antoine Martinus, 9032 Wondelgem (BE); SLEUTELS, Tomas Hubertus Johannes, 8911 MA Leeuwarden (NL); KUNTKE, Philipp, 8911 MA Leeuwarden (NL); HAMELERS, Hubertus Victor Marie, 8911 MA Leeuwarden (NL); BUISMAN, Cees Jan Nico, 8911 MA Leeuwarden (NL)
(74) Representative: Brantsandpatents bv
(86) International application number: PCT/NL2018/050680
(87) International publication number: WO 2019/078713

(56) References cited:
- WO-A1-2013/060331
- DE-A1-102007 031 688
- US-A1- 2013 065 285
- GANG LUO ET AL: "Integrated biogas upgrading and hydrogen utilization in an anaerobc reactor containing enriched hydrogenotrophic methanogenic culture", BIOTECHNOLOGY AND BIOENGINEERING, WILEY ETC , vol. 109, no. 11 28 May 2012 (2012-05-28), pages 2729-2736, XP002711228, ISSN: 0006-3592, DOI: 10.1002/BIT.24557 Retrieved from the Internet: URL:http://onlinelibrary.wiley.com/doi/10. 1002/bit.24557/pdf [retrieved on 2012-11-01]
- LU YUE ET AL: "Comparison analysis on the energy efficiencies and biomass yields in microbial CO2fixation", PROCESS BIOCHEMISTRY, vol. 62, 19 July 2017 (2017-07-19), pages 151-160, XP085228471, ISSN: 1359-5113, DOI: 10.1016/J.PROCBIO.2017.07.007
- STILLER M ET AL: "Hydrogenase activity in Chlorella", BIOCHIMICA ET BIOPHYSICA ACTA (BBA) - GENERAL SUBJECTS, ELSEVIER, AMSTERDAM, NL, vol. 93, no. 1, 9 October 1964 (1964-10-09), pages 174-176, XP025793151, ISSN: 0304-4165, DOI: 10.1016/0304-4165(64)90274-0 [retrieved on 1964-10-09]

## Description

The invention relates to a method for the enhancement of (biomass) productivity of hydrogenotrophic micro-organisms. More specifically, the method uses hydrogenotrophic micro-organisms, such as bacteria and/or algae, for the production of biomass.

### Background

In general, biomass is produced under influence of light. For example, algae are produced and commercially applied in food products and feed.

Growth rate of the biomass is limited to the availability of the gases that are involved in the reactions to grow the biomass. This limited availability of required gaseous components is caused by the relatively low solubility of these relevant gasses and thereby the relatively low gas-to-liquid transfer rate. Also, in practice often valuable hydrogen gas is lost via the so-called bioreactor off-gas. When air is used as oxygen source, gas needs to be vented to prevent gas accumulation in the process. This reduces the efficiency of the biomass growth. Furthermore, there is a safety risk involved when using a mixture of hydrogen (H₂) and oxygen (O₂). This provides restrictions to the process that thereby hinder optimal biomass growth.

DE102007031688A1 describes a process for producing methane by means of the combined activity of algae, which are fed with CO₂ and produce H₂ and O₂; and methanogenesis bacteria which subsequently use H₂ to produce methane. The produced O₂ is bound in the algae and not further used.

An objective of the present invention is to provide a method for the enhancement of the biomass productivity and/or concentration that obviates or at least reduces the aforementioned problems and/or is more efficient as compared to conventional methods for producing biomass.

### Summary

The invention is set out in the appended set of claims 1 to 11.

### Description

The objective is achieved with the method for the enhancement of biomass productivity using hydrogenotrophic micro-organisms, the method comprising the steps of:
- providing a reactor comprising a reactor vessel, at least one gas inlet and/or hydrogen production system, at least one fluid inlet, and at least one fluid outlet;
- charging the reactor with at least one hydrogenotrophic micro-organism culture;
- providing a gas feed to the at least one gas inlet with the gas feed comprising an amount of hydrogen gas and/or producing hydrogen gas with the hydrogen production system;
- providing a fluid feed to the at least one fluid inlet;
- forming in-situ oxygen (O₂) by said phototrophic micro-organisms;
- removing and/or taking up of hydrogen (H₂), carbon dioxide (CO₂) and/or NH₃/NH₄⁺ using the in-situ formed oxygen by said hydrogen-oxidising micro-organism culture;
- and growing
wherein said in-situ formation of oxygen by said phototrophic micro-organisms is controlled with an in-situ oxygen controller and/or redox controller.

The reactor comprises one or more gas inlets, fluid inlets, fluid outlets, and preferably also gas outlets. This enables providing a gas feed to the at least one gas inlet with the gas feed comprising an amount of hydrogen gas, and providing a fluid feed and enabling fluid outflow. In addition to, or as an alternative for the gas inlet, a hydrogen production system can be used to produce in-situ the required hydrogen, for example with an electrode system that is provided within the reactor. Preferably, to prevent accumulation of gas in the reactor a gas outlet is provided. The hydrogenotrophic micro-organisms use the supplied hydrogen to enhance the biomass growth using light, either directly and/or indirectly through the forming of oxygen.

According to the disclosure, the method comprises the steps of charging the reactor with at least one phototrophic micro-organism culture and in-situ forming of oxygen (O₂) by the phototrophic micro-organisms.

Phototrophic micro-organisms may not only utilize the hydrogen themselves, they may also produce oxygen that can be used by hydrogen-oxidizing micro-organisms. This has the advantage that the oxidizing products (equivalents) are produced in the reactor itself. More specifically, in-situ formed oxygen is achieved using the phototrophic micro-organisms. Therefore, this method obviates the limitation of the gas-to-liquid transfer for oxygen. Therefore, this method improves the efficiency of the method for the enhancement of biomass productivity.

In addition, biomass production is in some cases also limited by light. So, in case of feeding gas under light limiting conditions, this light limitation can be reduced and the productivity or concentration can be increased.

Furthermore, generating oxygen in-situ also prevents dilution of hydrogen and/or oxygen by air or oxygen additions. This prevents dilution of hydrogen, thereby allowing for higher hydrogen partial pressure, which reduces the gas-to-liquid transfer limitation for hydrogen. Furthermore, in case air would be used this would introduce nitrogen gas into the system that would accumulate typically in the headspace of the reactor. Such accumulation is prevented by the in-situ formation of oxygen. This further enhances the biomass productivity.

In an embodiment of the invention, light energy is used to split water (H₂O) and assimilate carbon dioxide (CO₂), resulting in formation of carbohydrates ([CH₂O]) and oxygen (O₂):

a) H₂O + CO₂ → [CH₂O] + O₂

Any (phototrophic micro-)organism supplied with hydrogen is capable of oxidation of hydrogen in an environment where hydrogen and oxygen co-occur, via the so-called oxyhydrogen (Knallgas) reaction:

b) 2H₂ + O₂ → 2H₂O

This reaction does not require any additional energy source, because of the energy released by oxidation of the hydrogen. Therefore the energy can also be used for CO₂ reduction (fixation), e.g.:

c) 6H₂ + O₂ + 2CO₂ → 2[CH₂O] + 4H₂O

The aforementioned oxyhydrogen pathway is interesting and may occur in the dark using an inorganic carbon source.

Phototrophic micro-organisms are able to produce (in-situ) oxygen in the system. By carefully monitoring the chemical balance in the system (e.g. oxygen concentration, redox potential and/or pH), the light energy (and/or: the CO₂ supply) can be adjusted such that there is just enough phototrophic oxygen production within the system (reaction a) to react with hydrogen (reaction b/c). Although the net stoichiometry will be comparable to photoreduction,

d) 2H₂ + CO₂ → H₂O + [CH₂O],

the interesting aspect of this approach is that it can occur under higher light intensities (i.e, higher reaction rates) and that the O₂-producing phototrophs can be different organisms than the hydrogen-oxidisers, and even physically separated from each other within the reactor. In principle, the use of a different co-culture, of (photoautotrophic) micro-organisms, such as microalgae, and (chemoautotrophic) hydrogen-oxidising micro-organisms, such as bacteria is thus possible in accordance to the present invention. This regulating principle can be applied to any process that requires partial aerobic/anaerobic conditions and/or particular redox state or gradient, such as sulphide oxidation reactors (oxidation of sulphide to elemental sulphur: CO₂ + 2H₂S → [CH₂O] + H₂O + 2S [avoiding complete oxidation to sulphate]); anammox (ANaerobic AMMonium OXidation) reactors (partial nitrification [nitritation]):

e. NH₄⁺ + 2O₂ → NO₂ + 2H₂O

f. NH₄⁺ + NO₂⁻ → N₂ + 2H₂O;

aerobic granular reactors, e.g. Nereda or reactors designed for degradation of organic (micro)pollutants over a redox gradient; and bioelectrochmical systems (i.e. microbial fuel cells, microbial synthesis cells, etc.).

Therefore, the method according to the present invention reduces the growth limitation used to the gas-to-liquid transfer rates, preferably prevents dilution and loss of valuable gasses, and provides a more safe process by the in-situ formation of oxygen.

These advantages are further improved when the method is performed in a closed system in one of the embodiments of the invention. This enables further control of the process steps.

The method comprises the step of controlling the in-situ formation of oxygen by the phototrophic micro-organisms with an in-situ oxygen controller and/or redox controller.

The in-situ oxygen controller may enable control of the in-situ production of oxygen. In preferred embodiments this controller regulates the in-situ oxygen concentration by controlling one or more of: light intensity, CO₂ supply, pH, redox potential. This enables direct in-situ oxygen production when required at a level that is beneficial for the biomass productivity. This further enhances the biomass productivity using the phototrophic micro-organisms.

The removing and/or taking up of CO₂ and/or NH₃/NH₄⁺ that uses the in-situ formed oxygen comprises the step of providing a hydrogen-oxidizing micro-organism culture for performing the hydrogenotrophic metabolic pathway.

Providing the hydrogen-oxidizing micro-organism culture, such as bacteria, enables biomass productivity by removal and/or uptake of the supplied CO₂ and/or NH₃/NH₄⁺. This improves the overall performance of the method.

Preferably, the hydrogen oxidizing micro-organisms are at least partially separated from the phototrophic micro-organisms. This may involve providing the hydrogen-oxidizing micro-organisms in a separate compartment or separate reactor from the phototrophic micro-organisms. This enables designing the compartments or reactors specifically for the type of micro-organisms.

Preferably, the hydrogen-oxidizing micro-organisms comprise chemo-autotrophic hydrogen-oxidizing bacteria.

In a presently preferred embodiment of the invention, the phototrophic micro-organisms comprise photo-autotrophic bacteria.

Phototrophic micro-organisms, especially phototrophic bacteria including photo-autotrophic bacteria use energy, such as light energy, to form carbohydrates using in-situ formed oxygen. This is preferably achieved in a so-called photo-bioreactor or another illuminated bioreactor, lighting is preferably achieved by providing one or more of externally lit flat panel photo-bioreactors, externally lit tubular photo-bioreactor, internally lit fluidized light sources, internally lit illuminated strings of LED's and/or internally lit quantum-dot coated materials such as beads on membranes. These and/or other appropriate energy sources enable the enhancement of biomass productivity.

The light spectrum in the reactor is preferably tuned specifically to the absorption properties of the taxa being cultivated, such as red light for organisms mainly dependent on chlorophyll adsorption (e.g., green (micro)algae), orange light for organisms with a large proportion of phycocyanin in the light-harvesting complex (e.g. many cyanobacteria), green light for organisms with phycoerythrin in their light-harvesting complex (e.g., certain cyanobacteria and red algae) or near-infrared light for organisms with bacteriochlorophyll absorption maxima in this part of the spectrum (e.g. many photosynthetic bacteria).

The phototrophic micro-organisms in one of the presently preferred embodiments of the invention belong to one or more of the following taxa of phototrophic bacteria:
- Purple sulfur bacteria (Chromatiaceae), such as *Ectothiorhodospira*, *Chromatium, Thiocapsa*, *Thiospirillum, Thiodictyon*, and/or *Thiopedia*;
- Purple non-sulfur bacteria (Ectothiorhodospiraceae, formerly Rhodospirillaceae), such as *Rhodomicrobium, Rhodobacter, Rhodopseudomonas*, *Rhodospirillum,* and/or *Rhodocyclus.*
- Green sulfur bacteria (Chlorobiaceae), such as *Chlorobium, Prostheochloris, Pelodictyon,* and/or *Chloroherpeton;*
- Green non-sulfur bacteria (Chloroflexaceae), such as *Chloroflexus;*
- Heliobacteria, such as *Heliobacterium, Helophilum,* and/or *Heliobacillus;*
- Cyanobacteria (Cyanofyta, Cyanophyceae), such as *Gloeothece, Synechococcus*, *Gloeocapsa*, *Gloeobacter, Dermocapsa*, *Synechocystis, Plectonema, Xenococcus, Pleurocapsa*, *Spirulina, Arthrospira*, *Oscillatoria*, *Limnothryx*, *Phormidium*, *Lyngbya*, *Cylindrospermum, Anabaena*, *Pseudanabaena*, *Nodularia*, *Calothrix*, *Nostoc, Cyanothece*, *Stigonema*, *Cyanobotrytis*, *Westicella*, *Loriella*, and/or *Nostichopsis;*
- Prochlorofyta, such as *Prochloron* and/or *Prochlorothrix;*
- Photosynthetic bacteria from other taxa or yet unlcassified, such as *Porphyrobacter neustonensis, Roseobacter denitrificans, Erythromicrobium sibericus, Pseudomonas radiora*, and/or certain *Pseudomonas* species.

Alternatively or in addition thereto, the phototrophic micro-organisms may comprise one or more of the following eukaryotic microalgae:
- Green algae (Chlorophyceae); such as *Chlorella, Chlorococcum, Ettlia*, *Neochloris, Desmodesmus, Scenedesmus*, *Selenastrum, Chlamydomonas, Haematococcus, Tetraselmis,* and/or *Dunaliella*;
- Traustochytrids (Traustochytriaceae) such as *Aurantiochytrium*, *Schizochytrium, Thraustochytrium,* and/or *Ulkenia*;
- Diatoms (Bacillariophyceae) such as *Chaetoceros, Skeletonema*, *Thallassiosira*, *Amphora, Navicula*, *Nitzschia*, and/or *Phaeodactylum*;
- Dinoflagellates (Dinophyceae) such as *Cryptothecodinium*, *Gymnodinium*, and/or *Oxyrrhis;*
- Euglenophytes (Euglenophyceae) such as *Euglena;*
- Eustigmatophytes (Eustigmatophyceae) such as *Nannochloropsis* and/or *Ellipsoidon;*
- Raphidophytes (Raphidophyceae) such as *Heterosigma* and/or *Chattonella*;
- Gold algae (Chrysophyceae) such as *Dicrateria*, *Isochrysis*, and/or *Pavlova*;
- Red algae (Rhodophyceae) such as *Porphyridium*, *Galdieria*, and/or *Cyanidioschyzon.*

It will be understood that also combinations of micro-organisms can be applied. This includes combinations of species that are specifically mentioned in this application and also other alternative species, for example hydrogen oxidizing bacteria such as *Hydrogenobacter thermophilus, Hydrogenovibrio marinus,* and *Helicobacter pylori,* and/or including non-phototrophic micro-organisms such as:
- Ammonium-oxidising and/or denitrifying bacteria, such as *Nitrosomonas, Nitrosococcus, Nitrobacter, Thiobacillus, Micrococcus,* and/or *Pseudomonas.*
- Anaerobic ammonium oxidising (Anammox) bacteria, such as *Anammoxoglobulus*, *Brocadia*, *Jettenia*, *Kuenenia*, and/or *Scalindua.*
- Other bacteria, archaea, fungi, protozoa and/or other (micro)organisms with useful metabolism and/or biomass (products).

It will be understood that combinations of embodiments can also be envisaged in accordance with the invention. Also, the method can be applied in a batch reactor or continuous reactor or a combination of a batch reactor and continuous reactor. In case the reactor involves a continuous culture, biomasses preferably retained by membrane or in granules, flocs or biofilms, optionally involving additional materials for immobilisation of the biomass. As was mentioned, the different processes that are illustrated can be performed in different compartments or in different reactors.

The present invention further relates to a system for the enhancement of biomass productivity using hydrogenotrophic micro-organisms according to the invention, with the system comprising:
- a reactor comprising a reactor vessel, at least one gas inlet and/or hydrogen production system, at least one fluid inlet, at least one fluid outlet, and preferably at least one gas outlet, with the gas inlet configured to supply an amount of hydrogen gas;
- at least one hydrogenotrophic micro-organism culture to enable performing the method in accordance to an embodiment of the present invention; and
the reactor vessel comprises a phototrophic micro-organism culture and an in-situ oxygen controller configured for controlling the in-situ oxygen production by the phototrophic micro-organisms.

The system provides the same effects and advantages as described for the method. In particular, the system is capable of performing the method of one of the embodiments of the invention.

Preferably, the reactor vessel comprises hydrogen-oxidizing micro-organisms.

In a further presently preferred embodiment, the reactor vessel comprises a first vessel compartment for the phototrophic micro-organism culture at the second vessel compartment for the hydrogen-oxidizing/hydrogenothropic micro-organism culture. This enables a specific design of the individual compartments or vessels.

In a further preferred embodiment of the invention the system further comprises a light source.

Providing a light source enables influencing or manipulation of the reactions and reaction rates that take place in the vessel. The light source may involve external sources that are provided outside the reactor and internal sources that are provided inside the reactor including floating sources. Preferably, the system comprises a light source controller that is operatively connected to the in-situ oxygen controller. This enables control of the light source or light sources to control the in-situ oxygen production as determined by the in-situ oxygen controller.

Further advantages, features or details of the invention are elucidated on the basis of preferred embodiments thereof, wherein reference is made to the accompanying drawings, in which:
- Figure 1 illustrates an embodiment of a system with gas feeds for enhancing biomass growth according to the invention;
- Figures 2 and 3 show alternative embodiments of a system for enhancing biomass growth according to the invention with a photo-bioreactor;
- Figure 4 A-B shows biomass growth results; and
- Figure 5 shows some experimental results.

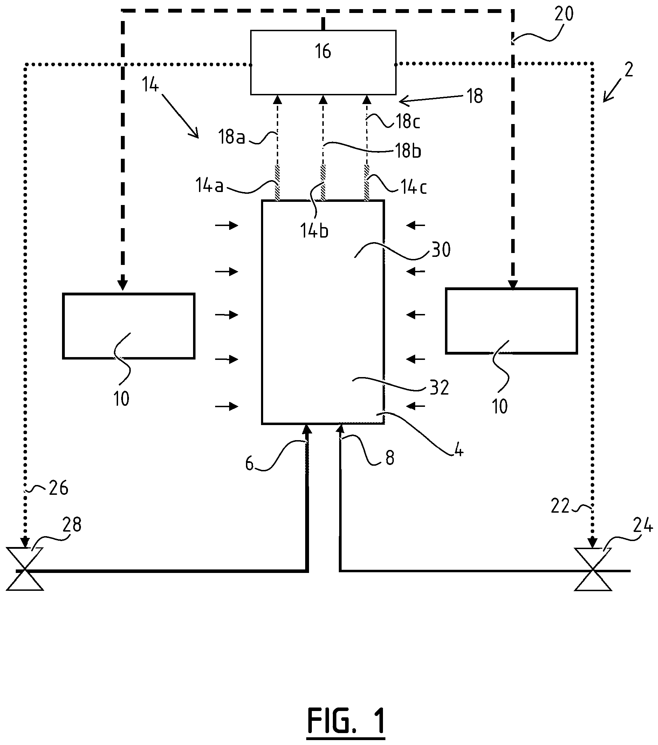

System 2 (figure 1) comprises reactor 4 having a first (hydrogen) inlet 6 and a second (CO₂) inlet 8. LED's 10 are provided around reactor 4 to provide light to reactor 4. It will be understood that light sources could also be provided in alternative embodiments, including floating LED's inside the reactor. Reactor 4 further comprises electrode system 14 with pH electrode 14a, redox electrode 14b and DO electrode 14c. Controller 16 receives and/or sends measurement/control signals 18, 18a, 18b, 18c to the respective electrodes 14. In use, controller 16 sends control signals 20 to LED's 10 and control signal(s) 22, 26 to control valves 24, 28 that are associated to respective inlets 6, 8. Reactor 4 further comprises phototrophic micro-organisms 30 and hydrogen-oxidising micro-organisms 32.

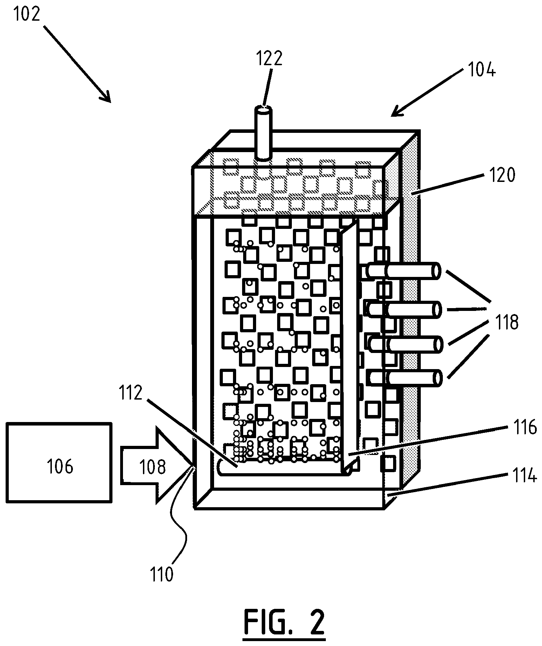

Alternative system 102 (figure 2) comprises reactor 104 and gas system 106 for gas mixing and optionally humidification. Gas system 106 produces gas mixture 108 that is provided to inlet 110 of reactor 104. In use, gas sparger 112 provides gas in reactor 104. In the illustrated embodiment transparent flat panel 114 is provided to enable easy monitoring the inside of reactor 104 that in use is filled with medium and micro-organisms. Reactor 104 further comprises a number of ports. For example, ports 118 can be used for medium addition, sampling, sensoring and harvesting. In the illustrated embodiment back panel 120 is provided that contains (LED) light sources and optionally an integrated temperature control. Gas outlet 122 is optionally provided with a condenser and/or gas analyser and/or gas recirculation.

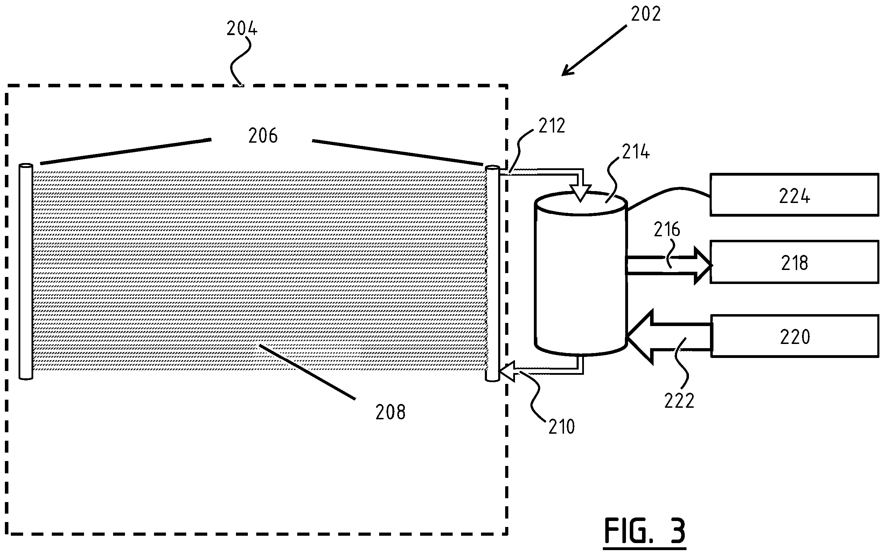

Another alternative system 202 (figure 3) comprises reactor 204 with tube supports 206 and transparent flow-through tubes 208 that in use are filled with culture medium and micro-organisms. Tube supports 206 preferably comprise manifolds. Reactor 204 is provided with inlet 210 and outlet 212 that are connected to vessel 214 for monitoring and control, medium addition, and gas exchange. Biomass harvest port 216 enables harvesting biomass that is produced and provides it to system 218 for sampling, harvesting and further processing. Gas system 220 mixes gases and optionally comprises a gas analyser. Gas system 220 provides a gas mixture to inlet port 222. Controller 224 is connected to sensors to monitor and control the operation.

It will be understood that different features of the illustrated embodiments can be exchanged, such as electrode system 18, back panel 120, vessel 214 and/or any other illustrated feature.

To circumvent hydrogenotrophic growth limitations systems 2, 102, 202 preferably comprise a hydrogen-fed bioreactor and a photo-bioreactor, reactors that are optionally integrated, so that the oxidizing equivalents are generated by phototrophs like microalgae. The phototrophs in reactor 4, 104, 204 could utilize the hydrogen themselves or produce oxygen that is used by hydrogen oxidizers. According to the preferred embodiments of the invention the oxidizing equivalents are produced in the reactor liquor. Gas-to-liquid transfer limitation for oxygen will therefore no longer be an issue. Because in the preferred embodiments oxygen is generated in situ in reactor 4, 104, 204, the hydrogen is not diluted by air or oxygen additions. Air would introduce excess gas that could accumulate in the headspace of the reactor. The in situ production of pure oxygen thus prevents the dilution of hydrogen, allowing for a high hydrogen partial pressure, which reduces the gas-to-liquid transfer limitation for hydrogen. Preferably, controller 16 keeps the oxygen partial pressure in reactor 4, 104, 204 in the safe range by controlling the oxygen production. This is advantageous because the hydrogen feeding is often not a suitable control parameter since the hydrogen flow is typically produced elsewhere by an independent process can thus not be easily reduced or interrupted without losing valuable hydrogen. Optionally, the oxygen concentration is kept in the safe range by reducing the light intensity and/or CO₂ feeding for photosynthesis using the oxidation/reduction potential or redox potential, the dissolved oxygen (DO) concentration, and/or the pH, for example.

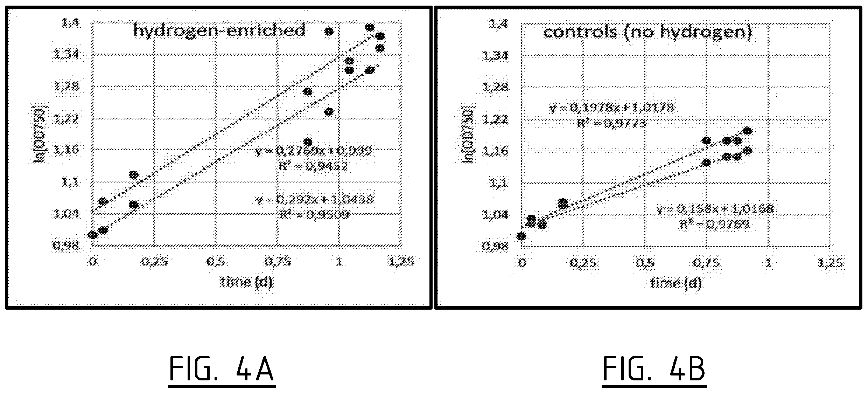

Experiments have been performed. In one of the experiments unicellar microalgae are applied that are commonly used as algae culture (*Chlorella sorokiniana*)*.* Results (see figure 4 A-B) show that under unlimited hydrogen supply, an increase in growth rate between 24% and 63% was observed as compared to growth rates in the controls (no additional hydrogen, only light as energy source). In the figures biomass density was expressed as the natural logarithm of the optical density measured at 750 nanometers and scaled to an initial value of 1 over time of *Chlorella sorokiniana* in a flat panel photobioreactor. Slopes of the regression lines represent exponential growth rates. This illustrates the operation according to the invention to enhance biomass growth and more particularly illustrates that "dirty" waste streams can be converted into clean feedstock for the culture of phototrophs. For example, this allows having complete control on the safety of the biomass, which can be cultured axenically and without the risk of undesirable (wastewater or flue gas-associated) contaminants.

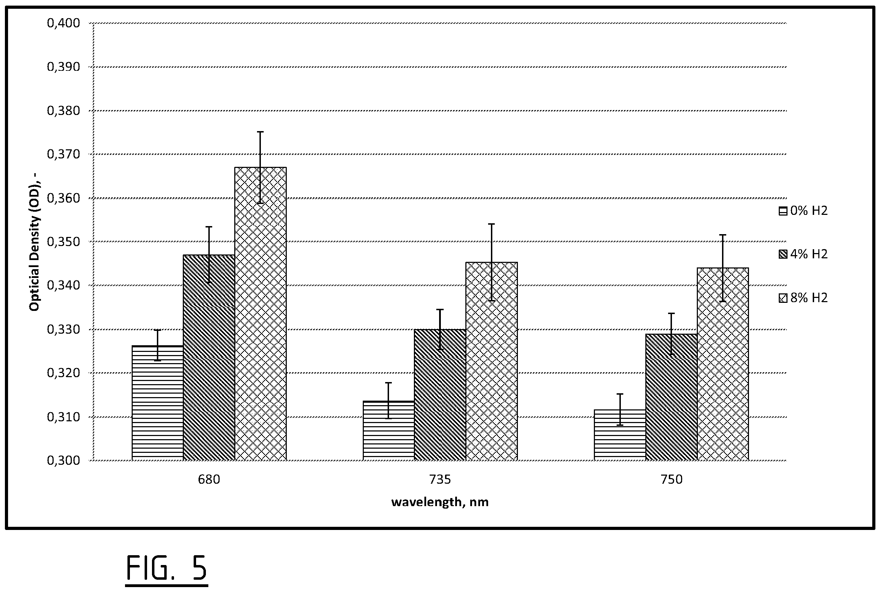

Experiments have been performed to investigate the growth effects of H₂ addition under light limiting conditions. In these experiments, micro algae (*Chlorella sorokiniana*) were cultivated in a photo bioreactor until light limiting conditions were reached and stable optical densities were measured. Afterwards, hydrogen gas was added to the system in two concentrations, 4 vol% and 8 vol%. The results showed that the optical density increased further with increasing hydrogen concentration. These results (figure 5) show that the addition of hydrogen under light limited condition results in a higher biomass production.

## Claims

1. Method for biomass production using hydrogenotrophic microorganisms, comprising the steps of:
- providing a reactor comprising a reactor vessel, at least one gas inlet and/or hydrogen production system, at least one fluid inlet, and at least one fluid outlet;
- charging the reactor with at least one phototrophic micro-organism culture and a hydrogen-oxidising micro-organism culture;
- providing a gas feed to the at least one gas inlet with the gas feed comprising an amount of hydrogen gas and/or producing hydrogen gas with the hydrogen production system;
- providing a fluid feed to the at least one fluid inlet;
- forming in-situ oxygen (O₂) by said phototrophic micro-organisms;
- removing and/or taking up of hydrogen (H2), carbon dioxide (CO2) and/or NH3/NH4+ using the in-situ formed oxygen by said hydrogen-oxidising micro-organism culture; and
- growing biomass;
wherein said in-situ formation of oxygen by said phototrophic micro-organisms is controlled with an in-situ oxygen controller and/or redox controller.

2. Method according to claim 1, wherein the method is performed in a closed system.

3. Method according to claim 1 or 2, wherein the in-situ oxygen controller regulates the in-situ oxygen concentration by controlling one or more of: light intensity, CO2 supply, pH, redox potential.

4. Method according to any one of the forgoing claims , wherein the hydrogen-oxidising micro-organisms are at least partially separated from the phototrophic micro-organisms.

5. Method according to any one of the forgoing claims, wherein the hydrogen-oxidising micro-organisms comprise chemoautotrophic hydrogen-oxidising bacteria.

6. Method according to one of the foregoing claims, wherein the phototrophic micro-organisms comprise photo-autotrophic bacteria.

7. Method according to one of the foregoing claims, wherein the phototrophic micro-organisms comprise eukaryotic microalgae.

8. System for the enhancement of biomass productivity using hydrogenotrophic microorganisms, the system comprising:
- a reactor comprising a reactor vessel, at least one gas inlet and/or hydrogen production system, at least one fluid inlet, at least one fluid outlet, and preferably at least one gas outlet, with the gas inlet configured to supply an amount of hydrogen gas;
- at least one hydrogenotrophic micro-organism culture to enable performing the method according to one of the foregoing claims; and
the reactor vessel comprises a phototrophic micro-organism culture and an in-situ oxygen controller configured for controlling the in-situ oxygen production by the phototrophic micro-organisms.

9. System according to claim 8, wherein the reactor vessel comprising a first vessel compartment for the phototrophic micro-organism culture and a second vessel compartment for the hydrogenotrophic micro-organism culture.

10. System according to claim 8 or 9, further comprising a light source.

11. System according to claim 10, further comprising a light source controller that is operatively connected to the in-situ oxygen controller.

## Patentansprüche

1. Verfahren zum Produzieren von Biomasse mit Hilfe hydrogenotropher Mikroorganismen, die folgenden Schritte umfassend:
- Bereitstellen eines Reaktors, der einen Reaktorkessel, mindestens einen Gaseinlass und/oder ein Wasserstoffproduktionssystem, mindestens einen Fluideinlass und mindestens einen Fluidauslass umfasst,
- Beladen des Reaktors mit mindestens einer Kultur fototropher Mikroorganismen und einer Kultur wasserstoffoxidierender Mikroorganismen,
- Bereitstellen einer Gaszufuhr zu dem mindestens einen Gaseinlass, wobei die Gaszufuhr eine Menge von Wasserstoffgas umfasst, und/oder Produzieren von Wasserstoffgas mit dem Wasserstoffproduktionssystem,
- Bereitstellen einer Fluidzufuhr zu dem mindestens einen Fluideinlass,
- Bilden von Sauerstoff (O₂) durch die fototrophen Mikroorganismen in situ,
- Entfernen und/oder Aufnehmen von Wasserstoff (H2), Kohlenstoffdioxid (CO2) und/oder NH3/NH4+ mit Hilfe des in situ gebildeten Sauerstoffs durch die Kultur wasserstoffoxidierender Mikroorganismen und
- Züchten von Biomasse,
wobei die in-situ-Bildung von Sauerstoff durch die fototrophen Mikroorganismen mit einer in-situ-Sauerstoff-Steuerung und/oder einer Redoxsteuerung gesteuert wird.

2. Verfahren nach Anspruch 1, wobei das Verfahren in einem geschlossenen System ausgeführt wird.

3. Verfahren nach Anspruch 1 oder 2, wobei die in-situ-Sauerstoff-Steuerung die in-situ-Sauerstoffkonzentration durch Steuern eines oder mehrerer des Folgenden regelt: Lichtintensität, CO2-Zufuhr, pH-Wert, Redoxpotential.

4. Verfahren nach einem der vorhergehenden Ansprüche, wobei die wasserstoffoxidierenden Mikroorganismen zumindest teilweise von den fototrophen Mikroorganismen getrennt sind.

5. Verfahren nach einem der vorhergehenden Ansprüche, wobei die wasserstoffoxidierenden Mikroorganismen chemoautotrophe wasserstoffoxidierende Bakterien umfassen.

6. Verfahren nach einem der vorhergehenden Ansprüche, wobei die fototrophen Mikroorganismen fototrophe Bakterien umfassen.

7. Verfahren nach einem der vorhergehenden Ansprüche, wobei die fototrophen Mikroorganismen eukaryotische Mikroalgen umfassen.

8. System zum Verbessern der Biomasseproduktion mit Hilfe hydrogenotropher Mikroorganismen, wobei das System Folgendes umfasst:
- einen Reaktor, der einen Reaktorkessel, mindestens einen Gaseinlass und/oder ein Wasserstoffproduktionssystem, mindestens einen Fluideinlass, mindestens einen Fluidauslass und vorzugsweise mindestens einen Gasauslass umfasst, wobei der Gaseinlass dafür gestaltet ist, eine Menge von Wasserstoffgas zuzuführen,
- mindestens eine Kultur hydrogenotropher Mikroorganismen, um das Ausführen des Verfahrens nach einem der vorhergehenden Ansprüche zu ermöglichen, und
wobei der Reaktorkessel eine Kultur fototropher Mikroorganismen umfasst und eine in-situ-Sauerstoff-Steuerung, die zum Steuern der in-situ-Sauerstoffproduktion durch die hydrogenotrophen Mikroorganismen konfiguriert ist.

9. System nach Anspruch 8, wobei der Reaktorkessel eine erste Kesselkammer für die Kultur fototropher Mikroorganismen und eine zweite Kesselkammer für die Kultur hydrogenotropher Mikroorganismen umfasst.

10. System nach Anspruch 8 oder 9, ferner eine Lichtquelle umfassend.

11. System nach Anspruch 10, ferner eine Lichtquellensteuerung umfassend, die funktionsfähig mit der in-situ-Sauerstoff-Steuerung verbunden ist.

## Revendications

1. Procédé de production de biomasse utilisant des micro-organismes hydrogénotrophes, comprenant les étapes consistant à :
- fournir un réacteur comprenant une cuve de réacteur, au moins une entrée de gaz et/ou un système de production d'hydrogène, au moins une entrée de fluide, et au moins une sortie de fluide ;
- charger le réacteur avec au moins une culture de micro-organismes phototrophes et une culture de micro-organismes oxydant l'hydrogène ;
- fournir une alimentation en gaz à l'au moins une entrée de gaz, l'alimentation en gaz comprenant une quantité d'hydrogène gazeux et/ou produire de l'hydrogène gazeux avec le système de production d'hydrogène ;
- fournir une alimentation en fluide à ladite au moins une entrée de fluide ;
- former de l'oxygène (O₂) in situ par lesdits micro-organismes phototrophes ;
- éliminer et/ou absorber l'hydrogène (H2), le dioxyde de carbone (CO2) et/ou le NH3/NH4+ en utilisant l'oxygène formé in situ par ladite culture de micro-organismes oxydant l'hydrogène ; et
- faire croître de la biomasse ;
dans lequel ladite formation d'oxygène in situ par lesdits micro-organismes phototrophes est contrôlée avec un contrôleur d'oxygène in situ et/ou un contrôleur redox.

2. Procédé selon la revendication 1, dans lequel le procédé est réalisé dans un système fermé.

3. Procédé selon la revendication 1 ou 2, dans lequel le contrôleur d'oxygène in situ régule la concentration d'oxygène in situ en contrôlant l'un ou plusieurs parmi : l'intensité lumineuse, l'apport de CO2, le pH, le potentiel redox.

4. Procédé selon l'une des revendications précédentes, dans lequel les micro-organismes oxydant l'hydrogène sont au moins partiellement séparés des micro-organismes phototrophes.

5. Procédé selon l'une des revendications précédentes, dans lequel les micro-organismes oxydant l'hydrogène comprennent des bactéries chimioautotrophes oxydant l'hydrogène.

6. Procédé selon l'une des revendications précédentes, dans lequel les micro-organismes phototrophes comprennent des bactéries photo-autotrophes.

7. Procédé selon l'une des revendications précédentes, dans lequel les micro-organismes phototrophes comprennent des microalgues eucaryotes.

8. Système destiné à améliorer la productivité de la biomasse à l'aide de micro-organismes hydrogénotrophes, le système comprenant :
- un réacteur comprenant une cuve de réacteur, au moins une entrée de gaz et/ou un système de production d'hydrogène, au moins une entrée de fluide, au moins une sortie de fluide, et de préférence au moins une sortie de gaz, l'entrée de gaz étant conçue pour fournir une quantité d'hydrogène gazeux ;
- au moins une culture de micro-organismes hydrogénotrophes pour permettre la mise en oeuvre du procédé selon l'une des revendications précédentes ; et
la cuve de réacteur comprend une culture de micro-organismes phototrophes et un contrôleur d'oxygène in situ conçu pour contrôler la production d'oxygène in situ par les micro-organismes phototrophes.

9. Système selon la revendication 8, dans lequel la cuve de réacteur comprend un premier compartiment de cuve pour la culture de micro-organismes phototrophes et un second compartiment de cuve pour la culture de micro-organismes hydrogénotrophes.

10. Système selon la revendication 8 ou 9, comprenant en outre une source lumineuse.

11. Système selon la revendication 10, comprenant en outre un contrôleur de source lumineuse qui est connecté de manière fonctionnelle au contrôleur d'oxygène in situ.
